# EUROPÄISCHE PATENTSCHRIFT

(11) **EP 0 901 779 B1**
(45) Veröffentlichungstag und Bekanntmachung des Hinweises auf die Patenterteilung: **06.08.2008**
(21) Anmeldenummer: 97118092.2
(22) Anmeldetag: 17.10.1997
(51) Int. Cl.: A61F 11/00

(54) **Anordnung zum Einstellen und Fixieren der Relativlage zweier Elemente eines aktiven oder passiven Hör-Implantats**
Device for adjusting and fixing the relative position of two elements of an active or passive hearing aid.
Dispositif permettant de régler et fixer la position relative de deux éléments d'une prothèse auditive active ou passive

(30) Priorität: 03.09.1997 DE 19738587
(43) Veröffentlichungstag der Anmeldung: 17.03.1999
(73) Patentinhaber: PHONAK AG, 8712 Stäfa (CH)
(72) Erfinder: Lehner, Rolf, Dipl.-Ing., 73734 Esslingen (DE); Leysieffer, Hans, Dipl.-Ing. Dr., 82024 Taufkirchen (DE)
(74) Vertreter: Schwan, Gerhard

(56) Entgegenhaltungen:
- US-A- 4 655 776
- US-A- 5 370 689
- US-A- 5 433 749

## Beschreibung

Die Erfindung betrifft eine Anordnung zum Einstellen der Relativlage zweier Elemente eines aktiven oder passiven Hör-Implantats und zum Fixieren dieser Elemente in der eingestellten Relativlage.

Aus EP-A-0 499 940 ist ein elektromechanischer Wandler für implantierbare Hörgeräte zur direkten mechanischen Anregung des Mittel- bzw. Innenohres bekannt, bei dem eine Wand eines hermetisch dichten und biologisch kompatiblen Gehäuses als schwingfähige Membran ausgeführt ist, die mit einer auf der Innenseite aufgebrachten piezoelektrischen Keramikscheibe ein elektromechanisch aktives Heteromorph-Verbundelement darstellt, und deren mechanische Schwingungen über eine auf der Außenseite der Membran fest fixierte, mechanisch steife Koppelstange und ein mechanisch steifes Koppelelement auf ein Mittelohr-Ossikel bzw. direkt auf das Innenohr übertragen werden.

Bei der bekannten Anordnung ist das Koppelelement an dem mit der Koppelstange zu verbindenden Ende als Bandschlaufe ausgebildet, die in eine Ausbuchtung der Koppelstange eingelegt ist und die im fixierten Zustand den ausgebuchteten Teil der Koppelstange auf einem Teil seines Umfangs umschlingt. Das Fixieren erfolgt durch Zudrücken der Bandschlaufe oder durch Applikation eines Klebstofftropfens. Die gegenseitige Lage von Koppelstange und Koppelelement in Achsrichtung der Koppelstange ist dabei durch die Ausbuchtung der Koppelstange fest vorgegeben, wobei die Ausbuchtung dazu beiträgt, das Koppelelement in der vorgesehenen Lage mit Bezug auf die Koppelstange zu halten. Gleichwohl kann die Langzeitstabilität der Verbindung von Koppelstange und Koppelelement zu wünschen übriglassen.

Der Erfindung liegt die Aufgabe zugrunde, eine Anordnung zu schaffen, die es erlaubt, die Relativlage zweier Elemente eines aktiven oder passiven Hör-Implantats auf einfache Weise am Implantationsort in situ einzustellen und die Elemente in der eingestellten Relativlage zuverlässig und langzeitstabil zu fixieren.

Diese Aufgabe wird erfindungsgemäß gelöst durch eine Anordnung zum Einstellen der Relativlage zweier Elemente eines aktiven oder passiven Hör-Implantats und zum Fixieren dieser Elemente in der eingestellten Relativlage, wobei eines der Elemente mindestens im Fixierbereich hülsenförmig ausgebildet und mittels eines Crimpwerkzeugs plastisch kaltverformbar ist, wobei das andere Element mindestens im Fixierbereich stabförmig ausgebildet, mit rauher Oberfläche versehen und unter dem Einfluß der mittels des Crimpwerkzeugs ausgeübten Crimpkraft nicht plastisch kaltverformbar ist, und wobei im fixierten Zustand der hülsenförmige Teil des einen Elements durch die Crimpkraft kaltfließend verformt auf dem stabförmigen Teil des anderen Elements spielfrei und dauerhaft befestigt ist.

Die Anordnung nach der Erfindung kommt ohne Fixierhilfe nach Art der Koppelstangen-Ausbuchtung der bekannten Lösung aus. Der stabförmige Teil kann vielmehr ohne weiteres geradlinig ausgebildet sein, so daß im Zuge der Implantation eine große Flexibilität für eine In-Situ-Einstellung der Relativlage der beiden Elemente nicht nur in Umfangsrichtung, sondern auch in Axialrichtung des stabförmigen Teils gewährleistet ist. Durch die kaltfließende Verformung des hülsenförmigen Teils auf dem rauhen stabförmigen Teil wird eine gegenseitige Verkrallung der beiden Teile erreicht, die in der Lage ist, selbst in Axialrichtung Kräfte zu übertragen, die um Zehnerpotenzen höher als die notwendigen Stimulationskräfte liegen. Dies dürfte insbesondere darauf zurückzuführen sein, daß die Axialkraftübertragung auf die Summe der Mikroverzahnungen zwischen der rauhen Oberfläche des stabförmigen Teils und dem kaltfließend verformten Werkstoff des hülsenförmigen Bauteils sowie auf einen zusätzlichen Reibkraftanteil zwischen den beiden Bauteilen zurückgeht. Die erfindungsgemäße Anordnung erlaubt es, das hülsenförmige Teil zunächst intraoperativ auf den stabförmigen Teil lose aufzustecken und dann die beiden Teile in die geeignete Relativlage zu bringen, worauf das Crimpen in situ im Mittelohr erfolgt.

Entsprechend einer Abwandlung der Erfindung kann die Anordnung zum Einstellen der Relativlage zweier Elemente eines aktiven oder passiven Hör-Implantats und zum Fixieren dieser Elemente in der eingestellten Relativlage auch so aufgebaut sein, daß eines der Elemente mindestens im Fixierbereich als zuziehbare Bandschlaufe ausgebildet ist, daß das andere Element mindestens im Fixierbereich geradlinig stabförmig ausgebildet ist und daß im fixierten Zustand der schlaufenförmige Teil des einen Elements den geradlinig stabförmigen Teil des anderen Elements über einen Winkel von mindestens 360° umschließt und durch Kraft- und/oder Formschluß auf dem stabförmigen Teil des anderen Elements spielfrei und dauerhaft befestigt ist.

Bevorzugte weitere Ausgestaltungen der Erfindung ergeben sich aus den Unteransprüchen.

Insbesondere kann eines der beiden Elemente eine Koppelstange sein, die mit einem aktiv schwingfähigen Teil eines implantierbaren Hörgerätewandlers verbunden ist, während das andere der beiden Elemente ein Koppelelement ist, das an die Ossikelkette oder die Perilymphe ankoppelbar ist.

Das Implantat kann aber auch als Total- oder Teilersatz für die Ossikelkette ausgebildet sein.

Bevorzugte Ausführungsbeispiele der Anordnung nach der Erfindung werden nachstehend anhand der beiliegenden Zeichnungen näher erläutert. Es zeigen:
- Fig. 1: in größerem Maßstab schematisch einen Hörgerätewandler mit Koppelstange,
- Fig. 2: in noch größerem Maßstab eine perspektivische Darstellung eines mit der Koppelstange des Hörgerätewandlers nach Fig. 1 verbundenen Koppelelements zum Ankoppeln des Hörgerätewandlers an den langen Amboßfortsatz,
- Fig. 3: eine Seitenansicht des Koppelelements nach Fig. 2,
- Fig. 4: eine Stirnansicht des Koppelelements nach Fig. 2,
- Fig. 5: eine Draufsicht auf das Koppelelement gemäß Fig. 2,
- Fign. 6, 7 und 8: eine Seitenansicht, eine Stirnansicht bzw. eine Draufsicht eines Koppelelements gemäß einer abgewandelten Ausführungsform,
- Fign. 9 und 10: eine Seitenansicht und eine Stirnansicht eines Koppelelements mit glockenförmigem Ankoppelteil,
- Fign. 11 und 12: eine Seitenansicht und eine Stirnansicht eines Koppelelements mit kolbenförmigem Ankoppelteil,
- Fign. 13, 14 und 15: schematische Darstellungen des Crimpvorganges und des dazu verwendeten Crimpwerkzeugs,
- Fig. 16: schematisch eine passive Totalprothese mit gegenseitiger Crimpverbindung zweier Prothesenteile,
- Fig. 17: eine schematische Darstellung einer passiven Teilprothese mit gegenseitiger Crimpverbindung zweier Prothesenteile,
- Fig. 18: eine abgewandelte Ausführungsform einer Anordnung zum Einstellen der Relativlage zweier Elemente eines Hör-Implantats und zum Fixieren dieser Elemente in der eingestellten Relativlage mit noch nicht zugezogener Bandschlaufe sowie
- Fign. 19 und 20: eine Stirnansicht und eine Draufsicht der Anordnung gemäß Fig. 18 mit zugezogener Bandschlaufe.

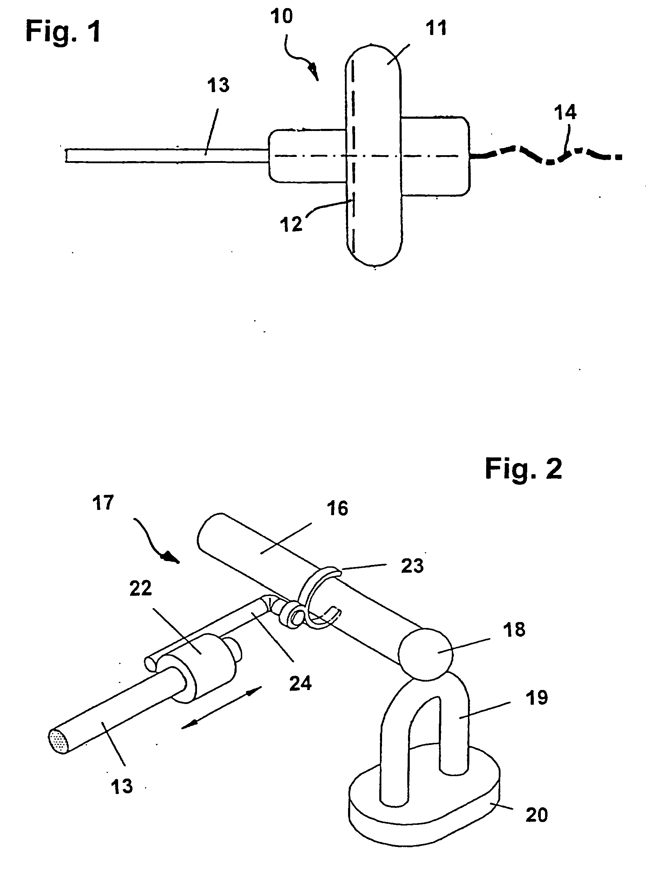

Fig. 1 zeigt in vergrößertem Maßstab einen implantierbaren Hörgerätewandler 10, der beispielsweise als Piezowandler zur vibratorischen Stimulation der Ossikelkette ausgelegt sein kann. Der Hörgerätewandler weist ein allseitig hermetisch dicht geschlossenes, biokompatibles Gehäuse 11 auf, und er kann mit einem elektromechanisch aktiven Heteromorph-Verbundelement der aus EP-A-0 499 940 bekannten Art ausgestattet sein. Mit einem in Fig. 1 nur schematisch angedeuteten aktiv schwingfähigen Teil 12 des Hörgerätewandlers 10 ist eine Koppelstange 13 mechanisch fest verbunden. Im veranschaulichten Ausführungsbeispiel hat die Koppelstange 13 im wesentlichen auf ihrer gesamten Länge die Gestalt eines geraden Zylinders. Wird an einen Kabelanschluß 14 des Hörgerätewandlers 10 eine elektrische Spannung angelegt, wird die Koppelstange 13 mittels des aktiv schwingfähigen Teils 12 zu vibratorischen Schwingungen in Axialrichtung der Koppelstange veranlaßt. Infolgedessen führen die von einem (nicht dargestellten) Mikrofon aufgenommenen und elektrisch gewandelten Audiosignale nach elektronischer Verstärkung in einem Elektronikmodul des Hörgeräts unmittelbar zu mechanischen Auslenkungen der Koppelstange 13. Diese Auslenkungen entsprechen der akustischen Information. Die Auslenkungen der Koppelstange 13 werden an die Ossikelkette des Mittelohrs bzw. an den Steigbügel oder das ovale bzw. runde Fenster oder auch ein artifizielles Fenster durch mechanische Koppelelemente weitergeleitet, die an der Koppelstange 13 fest fixiert werden können. Sie bewirken so bei entsprechender Auslegung des vorverarbeitenden elektronischen Systems den audiologischen Verstärkungseffekt.

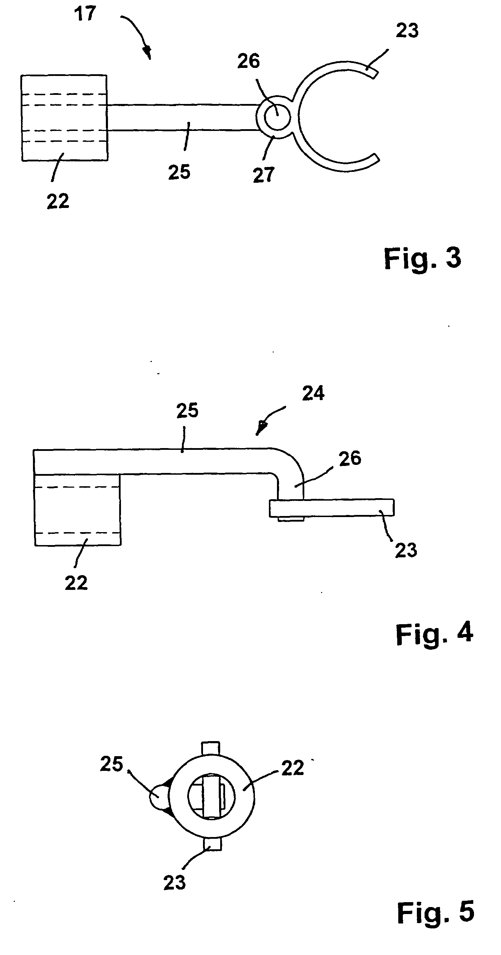

Fig. 2 zeigt in schematischer perspektivischer Darstellung ein zur Verbindung der Koppelstange 13 mit dem langen Amboßfortsatz 16 ausgelegtes Koppelelement 17. Eine solche Ankopplung eignet sich bei intakter Ossikelkette. Dabei sind in Fig. 2 das Amboß-Stapes-Gelenk 18, der Stapes-Überbau 19 und die Stapes-Fußplatte 20 schematisch dargestellt. Das Koppelelement 17 weist eine Crimphülse 22, ein C-förmiges Ankoppelteil 23 und ein Mittelteil 24 auf, welches für eine feste gegenseitige Verbindung von Crimphülse 22 und Ankoppelteil 23 sorgt. Das Koppelelement 17 ist in weiter vergrößertem Maßstab in den Fign. 3, 4 und 5 veranschaulicht. Wie dort gezeigt ist, besteht das Mittelteil 24 aus einem abgewinkelten Drahtstück, dessen einer Schenkel 25 langgestreckt und mit der Außenseite der Crimphülse 22 verbunden, z.B. verlötet oder verschweißt, ist. Der andere, kürzere Schenkel 26 des Mittelteils 24 greift mit seinem freien Ende in eine an dem Ankoppelteil 23 ausgebildete Öse 27 ein. Er ist dort beispielsweise durch Schweißen oder Löten befestigt. Im montierten Zustand umgreift das C-förmige Ankoppelteil 23 den langen Amboß-Fortsatz 16 (Fig. 2). Das C-förmige Ankoppelteil 23 kann insbesondere aus einer Reintitanfolie (Reinheit >99,6%) mit einer Dicke in der Größenordnung von 200 µm drahterodiert sein.

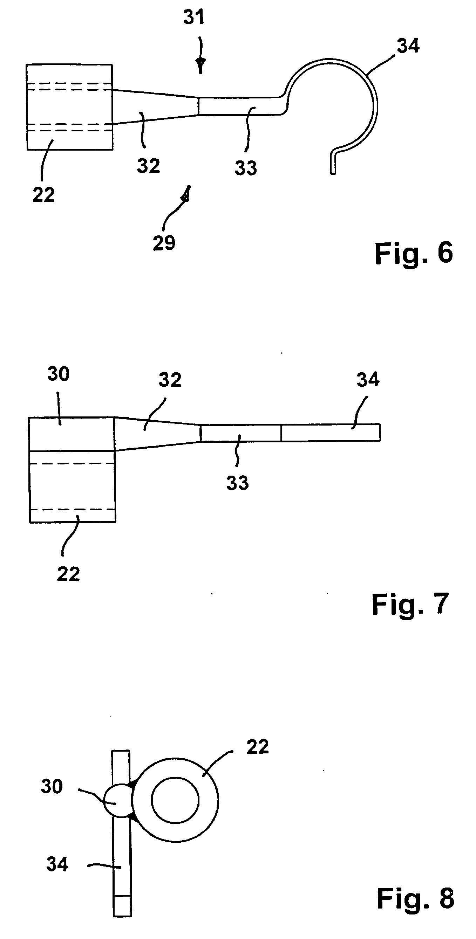

In den Fign. 6, 7 und 8 ist ein Koppelelement 29 entsprechend einer abgewandelten, ebenfalls insbesondere zur Ankopplung an dem langen Amboß-Fortsatz 16 geeigneten Ausführungsform gezeigt. Dabei steht die Crimphülse 22 in fester Verbindung (beispielsweise Schweiß- oder Lötverbindung) mit einem ersten zylindrischen Abschnitt 30 eines insgesamt mit 31 bezeichneten Mittelteils 31. An den zylindrischen Abschnitt 30 schließt ein sich konisch verjüngender Abschnitt 32 an, der in einen zweiten zylindrischen Abschnitt 33 übergeht. Dessen Durchmesser ist kleiner als der des Abschnitts 30. Der zylindrische Abschnitt 33 steht in einstückiger Verbindung mit einer Bandschlaufe 34, die beispielsweise um den langen Amboß-Fortsatz 16 herumgelegt werden kann.

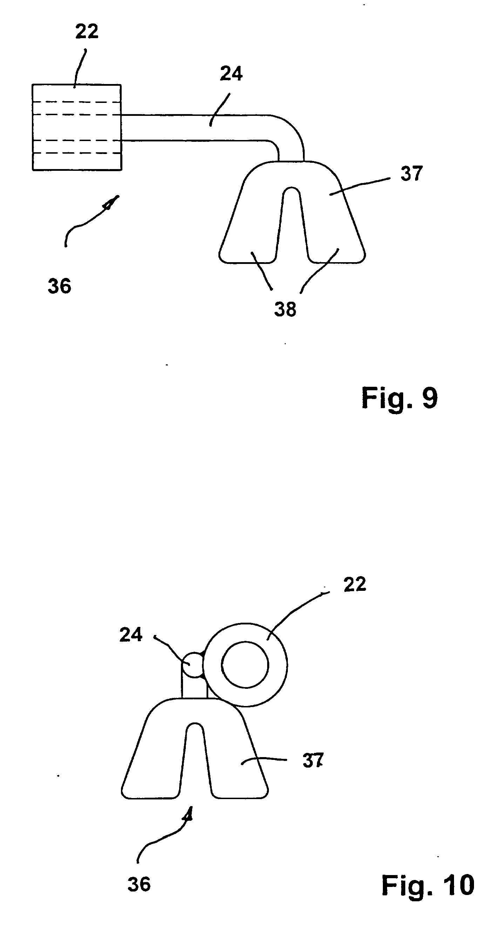

Die Fign. 9 und 10 zeigen ein weiter abgewandeltes Koppelelement 36. Dieses ist vor allem zur Ankopplung an das Stapes-Köpfchen bei fehlendem Amboß geeignet. Das Koppelelement 36 unterscheidet sich von dem Koppelelement 17 der Fign. 2 bis 5 im wesentlichen dadurch, daß an Stelle des C-förmigen Ankoppelteils 23 an dem Mittelteil 24 ein näherungsweise glockenförmiges Ankoppelteil 37 angebracht ist. Das Ankoppelteil 37 kann dem Goldglöckchen konventioneller Golddraht-BELL-Prothesen (siehe Steinbach, E.; Pusalkar, A.G.; Plester, D. (1990): Gehörknöchelchenersatz durch Goldprothesen. Zentralblatt HNO-Heilkunde 139:133) entsprechen. Das Ankoppelteil 37 läßt sich auf das Stapes-Köpfchen aufsetzen und dort durch Zusammendrücken der Glockenteile 38 fixieren.

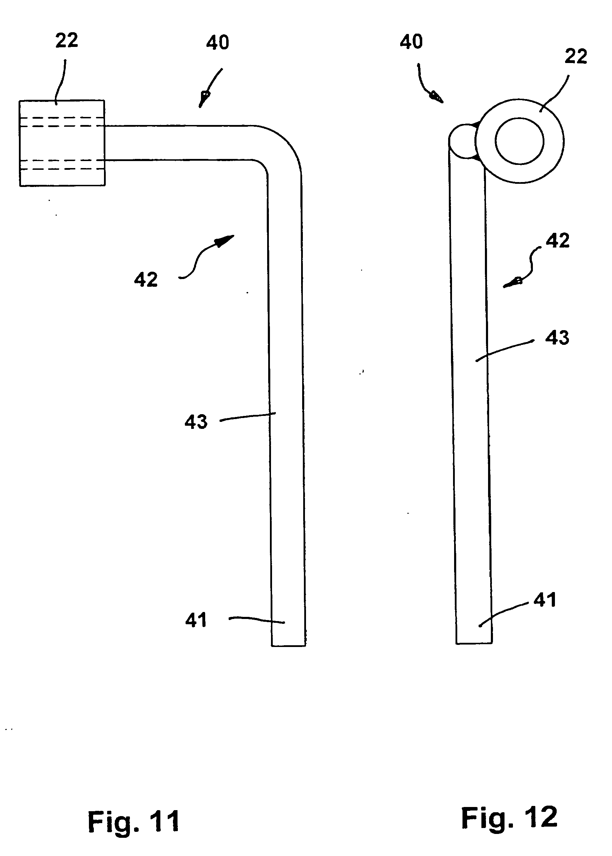

Bei fehlendem Steigbügelüberbau kann die Ankopplung des Hörgerätewandlers 10 direkt an die Perilymphe mittels eines Koppelelements 40 der in den Fign. 11 und 12 veranschaulichten Art erfolgen. Dabei ist ein kolbenförmiges Ankoppelteil 41 nach Art einer Stapes-Piston-Prothese vorgesehen, das über ein abgewinkeltes Mittelteil 42 mit der Crimphülse 22 fest verbunden ist und eine einstückige Verlängerung des von der Crimphülse 22 abliegenden Schenkels 43 des Mittelteils 42 darstellt. Zwecks Ankopplung an die Perilymphe kann die Koppelstange 13 als "künstlicher Amboß" durch den Chorda-Fazialis-Winkel ins Mittelohr geführt und deren distales Ende über der ovalen Nische positioniert werden. Nach Perforation der Fußplatte 20, z.B. mittels eines Erbium-YAG-Lasers, wird das Koppelelement 40 ähnlich wie bei einer Stapedektomie eingestellt und an der Koppelstange 13 befestigt. Das Ankoppelteil taucht dann über die Fußplattenperforation in das Vestibulum ein.

Die erwähnten Ankoppelteile sind ähnlich denjenigen von bewährten Standardelementen von Mittelohrprothesen, und sie bieten den Vorteil, daß sie mit gebräuchlichen Standard-OP-Instrumenten, wie zum Beispiel McGee-Zängchen, Maulzängchen, Doppellöffel oder Winkelhäkchen mechanisch geschlossen, aber im Bedarfsfall auch wieder geöffnet werden können.

Den zuvor beschriebenen Ausführungsformen ist gemeinsam, daß die Crimphülse 22 intraoperativ auf das distale Ende der Koppelstange 13 lose aufgesteckt, das Koppelelement axial und radial in die geeignete Position geschoben bzw. gedreht und die Hülse auf der Koppelstange in situ festgecrimpt werden kann.

Grundsätzlich kommt zwar auch ein externes Crimpen auf dem Operationstisch in Betracht. In der Regel verbietet sich dies jedoch, weil das Koppelelement naturgemäß erst gegen Ende der Implantation des Hörgerätes am Ossikel befestigt werden kann, da andernfalls ein erhöhtes Risiko einer Innenohrschädigung in Kauf genommen werden müßte. Außerdem besteht bei externem Crimpen die Gefahr, beim Einführen der Koppelstange das Koppelelement zu beschädigen. Das Koppelelement könnte dann nur mit erheblichem Aufwand wieder von der Koppelstange gelöst werden.

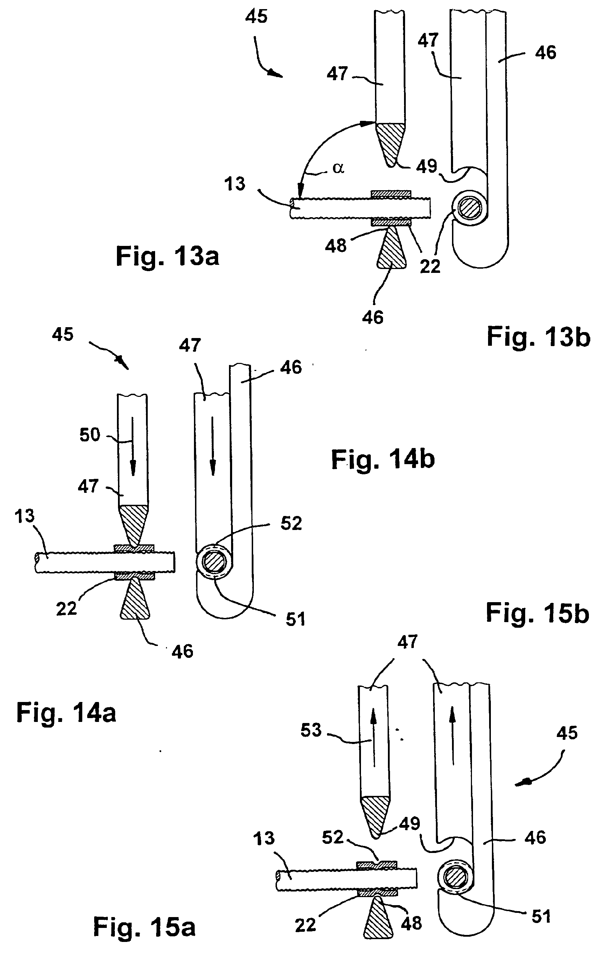

Zum Crimpen in situ eignet sich insbesondere eine an sich bekannte Hammerkopfstanze (z.B. STORZ 222800), die im Schneidenbereich in der aus den Fign. 13 bis 15 ersichtlichen Weise modifiziert wurde. Die so dem vorliegenden Zweck angepaßte Crimpzange weist ein stationäres Teil 46 und ein mit Bezug auf das stationäre Teil verschiebbares Teil 47 auf. Die Crimpzangenteile 46 und 47 sind mit einander gegenüberstehenden bogenförmigen Schneiden 48 bzw. 49 ausgestattet, deren Krümmungsradius dem Radius der Crimphülse 22 angepaßt ist., wobei vorzugsweise der Krümmungsradius der Schneiden etwas größer als der Außenradius der Crimphülse ist.

Zum Befestigen der Crimphülse 22 kann die Schneide 48 des stationären Crimpzangenteils 46 gegen die in die Sollposition gebrachte Crimphülse 22 entsprechend den Fign. 13a und 13b angelegt werden. Dann wird die Schneide 49 durch Verschieben des Crimpzangenteils 46 in Richtung des Pfeils 50 auf die Schneide 48 des stationären Crimpzangenteils 46 zubewegt. Durch Aufbringen einer entsprechenden Crimpkraft wird die Crimphülse 22 mittels der Schneiden 48, 49 im Bereich von zwei einander diametral gegenüberliegenden, bogenförmigen Crimpzonen 51, 52, die sich in Umfangsrichtung der Crimphülse 22 erstrecken, kaltfließend verformt (Fign. 14a und 14b). Dann wird durch Verschieben des Crimpzangenteils 47 in Richtung des Pfeils 53 die Schneide 49 von der Schneide 48 wegbewegt (Fign. 15a und 15b), worauf die Crimpzange 45 aus dem Mittelohrbereich herausgebracht wird.

Bei dem erläuterten Crimpvorgang wird vorzugsweise mit einem Crimpwinkel α (Fig. 13a) von näherungsweise 90° gearbeitet. Der Crimpwinkel α entspricht intraoperativ dem Winkel zwischen dem äußeren Gehörgang, durch den die Crimpzange 45 enaural ins Mittelohr eingeführt wird, und der Längsachse der Koppelstange 13. In der Praxis kann es vorkommen, daß die Koppelstange 13 mit der Crimpzange 45 nicht zuverlässig rechtwinklig, sondern nur etwas schräg zu erreichen ist. Der Schneidenbereich der Crimpzange 45 ist daher vorzugsweise so gestaltet, daß auch ein schräges Crimpen im Winkelbereich α = 90° ± 15° sicher gelingt. Zweckmäßig ist die Crimpzange 45 ferner mit einem (im einzelnen nicht dargestellten) mechanischen Überlastschutz zur Vermeidung übermäßig großer Bedienkräfte versehen.

Für eine dauerhafte und spielfreie Befestigung der Crimphülse 22 auf der Koppelstange 13 ist es wesentlich, daß die Crimphülse mittels des Crimpwerkzeugs (insbesondere der Crimpzange 45) plastisch kaltverformbar ist, während die Koppelstange 13 mindestens im Fixierbereich eine rauhe Oberfläche aufweist und unter dem Einfluß der mittels des Crimpwerkzeugs ausgeübten Crimpkraft nicht plastisch kaltverformbar ist. Eine gemittelte Rauhtiefe Rz von mindestens 10 µm ist für die rauhe Außenfläche der Koppelstange 13 im Bereich der Verbindung mit der Crimphülse 22 besonders zweckmäßig.

Die Crimphülse 22 besteht vorzugsweise aus Gold, insbesondere aus Feingold mit einem Goldgehalt > 99,99%, das vorzugsweise weichgeglüht wird. Das Weichglühen kann zweckmäßig über etwa 10 Minuten bei einer Temperatur in der Größenordnung von 500°C erfolgen. Ein solches weichgeglühtes Feingold kann eine Wickershärte HV von beispielsweise etwa 20 haben. Durch die mittels der Schneiden 48, 49 beidseits aufgebrachten hohen Preßkräfte wird ein Teilbereich der Crimphülse 22 in die rauhe Oberfläche der Koppelstange 13 eingedrückt. Nach dem Crimpen bleibt die Hülse 22 plastisch verformt, d.h. sie federt nicht mehr in ihre ursprüngliche Lage zurück, wie dies insbesondere in Fig. 15a angedeutet ist. Im Bereich der Krafteinleitungszonen kommt es auf diese Weise zu einer Mikroverzahnung zwischen der Crimphülse 22 und der Koppelstange 13. Eine solche Mikroverzahnung führt zusammen mit dem zusätzlich auftretenden Reibkraftanteil zu übertragbaren Axialkräften im Bereich von beispielsweise mindestens etwa 10 N. Diese Kräfte liegen um Zehnerpotenzen höher als die notwendigen Stimulationskräfte.

Für das Hör-Implantat soll eine möglichst geringe Anzahl unterschiedlicher Biometalle verwendet werden, um eine unerwünschte Potentialbildung entsprechend der elektrochemischen Spannungsreihe auszuschließen oder zu minimieren. Vorzugsweise besteht daher nicht nur die Crimphülse 22, sondern das gesamte Koppelelement 17, 29, 36 bzw. 40 (mit Ausnahme des C-förmigen Ankoppelteils 23) aus Feingold. Für diesen Werkstoff sind die Biokompatibilität und Langzeitstabilität speziell auch für Mittelohrräume ausreichend belegt. Darüberhinaus ist eine postoperative Lösbarkeit der Ossikelankopplung auch noch nach mehrjähriger Körperverweilzeit gegeben. Eine solche Lösung der Verbindung ist mit adäquatem Kraftaufwand und unter Verwendung von ohrchirurgischen Standardinstrumenten möglich. Die Anbindung des Koppelelements 17, 19, 36, 40 an das jeweilige Zielossikel oder die Perilymphe kann dabei in Anlehnung an bewährte Standardelemente gebräuchlicher passiver Mittelohrprothesen geschehen.

Es versteht sich jedoch, daß für das Koppelelement und insbesondere auch die Crimphülse 22 andere Werkstoffe gleichfalls in Frage kommen, so vor allem Platin, insbesondere Reinplatin, oder Silber, oder Legierungen von Gold, Platin bzw. Silber.

Für die Koppelstange 13 eignet sich vor allem implantierfähiges Titan, insbesondere Reintitan mit einer Reinheit > 99,6 %. Daneben kommen Platin, Niob oder Tantal oder Legierungen von Titan, Platin, Niob bzw. Tantal in Betracht. Gegebenenfalls kann die Koppelstange 13 aber auch aus einem implantierbaren keramischen Werkstoff, insbesondere Aluminiumoxid, bestehen.

Bei der Materialauswahl und der konstruktiven Gestaltung ist darauf zu achten, daß es darauf ankommt, den vibratorischen Stimulus (Aktio) des Wandlers 10 möglichst verlustfrei, d.h. schallhart, in das Zielossikel oder die Perilymphe einzuleiten. Dabei soll die Gesamtmasse der Koppelelemente vorzugsweise unter der Masse des Amboß liegen, die durchschnittlich 25 mg beträgt. Ein geringstmögliches Gewicht der Koppelelemente führt zudem zur Reduzierung von Trägheitskräften bei Beschleunigung durch externe Einflüsse wie Schlag, Vibration usw.

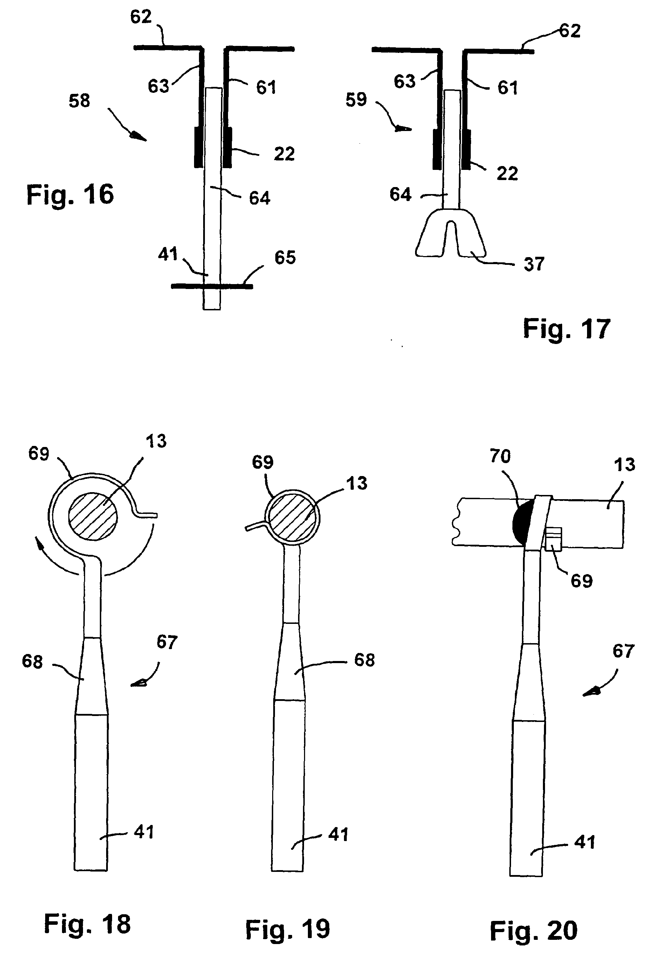

Die vorstehend im einzelnen erläuterte Kaltflußverbindung läßt sich auch für Elemente eines passiven Hör-Implantats vorsehen, das als Total- oder Teilersatz für die Ossikelkette ausgebildet ist. Ein Ausführungsbeispiel für eine Totalprothese ist schematisch in Fig. 16 bei 58 dargestellt, während Fig. 17 in ähnlicher schematischer Darstellung ein Beispiel einer Teilprothese 59 zeigt. Die Totalprothese 58 weist ein Verbindungselement 61 mit einem trommelfellseitigen Ankoppelteil 62 zum Ankoppeln der Prothese an das Trommelfell und einem damit fest verbundenen hohlen Zylinderteil 63 auf. Der Zylinderteil 63 geht an seinem von dem Ankoppelteil 62 abliegenden Ende in die Crimphülse 22 über. Zu dem Implantat 58 gehört ferner eine in die Crimphülse 22 und gegebenenfalls den Zylinderteil 63 eintauchende Koppelstange 64. Die Koppelstange 64 ist mindestens im Fixierbereich in der oben für die Koppelstange 13 erläuterten Weise mit einer rauhen Oberfläche versehen, und sie geht im veranschaulichten Ausführungsbeispiel einstückig in das kolbenförmige Ankoppelteil 41 über, das über eine Öffnung der Stapes-Fußplatte 20 in die Perilymphe eintaucht. Das Ankoppelteil 41 trägt an seinem von der Crimphülse 22 abliegenden Ende einen zum Beispiel ringförmigen Anschlag 65 zum Begrenzen der Eintauchtiefe des Ankoppelteiles 41.

Die Teilprothese gemäß Fig. 17 unterscheidet sich von der Totalprothese der Fig. 16 im wesentlichen nur dadurch, daß das von dem Ankoppelteil 62 abliegende Ende der Koppelstange ·64 mit dem glockenförmigen Ankoppelteil 37 verbunden ist, das, wie oben beschrieben, auf das Stapes-Köpfchen aufgesetzt werden kann.

Bei der Implantation der Prothesen 58 und 59 lassen sich das Verbindungselement 61 und die Koppelstange 64 analog der zuvor erläuterten Weise gegenseitig drehen und in Axialrichtung verstellen sowie durch Crimpen spielfrei und dauerhaft miteinander verbinden. Auf diese Weise können einfach und rasch patientenindividuell die Länge der Total- oder Teilprothese sowie, falls erforderlich, auch die gegenseitige Winkelausrichtung der Ankoppelteile 62 und 41 beziehungsweise 37 intraoperativ eingestellt werden.

Die Fign. 18, 19 und 20 zeigen eine weiter abgewandelte Ausführungsform einer Anordnung zum Einstellen der Relativlage zweier Elemente eines Hör-Implantats und zum Fixieren dieser Elemente in der eingestellten Relativlage. Dabei weist ein Koppelelement 67 das kolbenförmige Ankoppelteil 41 gemäß den Fign. 11, 12 und 16 und eine damit über ein Zwischenglied 68 verbundene Bandschlaufe 69 auf, die um die Koppelstange 13 herum zugezogen werden kann. In dem in den Fign. 19 und 20 veranschaulichten fixierten Zustand umschließt die Bandschlaufe 69 die Koppelstange 13 über einen Winkel von mindestens 360°, um durch Kraft- und/oder Formschluß für eine Festlegung der Bandschlaufe 69 mit Bezug auf die Koppelstange 13 in der jeweils eingestellten Position zu sorgen. Für einen Formschluß kann insbesondere dadurch gesorgt werden, daß nicht nur die Koppelstange 13, sondern auch die Bandschlaufe 69 an ihrer der Koppelstange zugewendeten Seite aufgerauht ist, so daß es beim Zuziehen der Bandschlaufe 69 zu einer gegenseitigen Mikroverzahnung zwischen Bandschlaufe und Koppelstange kommt. Gegebenenfalls kann für eine zusätzliche Sicherung durch Stoffschluß gesorgt sein, beispielsweise indem in der in Fig. 20 schematisch angedeuteten Weise eine kleine Menge an chirurgischem Knochenzement 70 aufgebracht wird.

Es versteht sich, daß bei den Ausführungsbeispielen nach den Fign. 11, 12 und 18 bis 20 an Stelle des kolbenförmigen Ankoppelteils 41 auch das C-förmige Ankoppelteil 23 oder die Bandschlaufe 34 oder das glockenförmige Ankoppelteil 37 vorgesehen sein können. In entsprechender Weise läßt sich bei der Teilprothese gemäß Fig. 17 das glockenförmige Ankoppelteil 37 durch das C-förmige Ankoppelteil 23 oder die Bandschlaufe 34 ersetzen. Auch bezüglich anderer Merkmale lassen sich die beschriebenen Anordnungen ohne weiteres variieren. Beispielsweise können mittels der Crimpzange 45 zwei oder mehr Crimpzonen nebeneinander in aufeinanderfolgenden Crimpvorgängen ausgebildet werden, oder das Crimpwerkzeug kann mit zwei oder mehr nebeneinanderliegenden Schneidenpaaren ausgestattet sein. Es ist unter anderem auch möglich, eine der beiden Schneiden 48, 49 durch ein amboßartiges Gegenstück zu ersetzen und nur eine der Crimpzonen 51 bzw. 52 auszubilden.

## Patentansprüche

1. Anordnung zum Einstellen der Relativlage zweier Elemente eines aktiven oder passiven Hör-Implantats (10, 58, 59) und zum Fixieren dieser Elemente in der eingestellten Relativlage, wobei eines der Elemente (17, 29, 36, 40, 61) mindestens im Fixierbereich hülsenförmig ausgebildet und mittels eines Crimpwerkzeugs (45) plastisch kaltverformbar ist, wobei das andere Element (13, 64) mindestens im Fixierbereich stabförmig ausgebildet, mit rauher Oberfläche versehen und unter dem Einfluß der mittels des Crimpwerkzeugs (45) ausgeübten Crimpkraft nicht plastisch kaltverformbar ist, und wobei im fixierten Zustand der hülsenförmige Teil des einen Elements durch die Crimpkraft kaltfließend verformt auf dem stabförmigen Teil des anderen Elements spielfrei und dauerhaft befestigt ist.

2. Anordnung nach Anspruch 1, **dadurch gekennzeichnet, daß** der Crimpwinkel (α) 90° ± 15° beträgt.

3. Anordnung nach Anspruch 1 oder 2, **dadurch gekennzeichnet, daß** zwei einander diametral gegenüberliegenden Crimpzonen (51, 52) vorgesehen sind.

4. Anordnung nach einem der vorhergehenden Ansprüche, **dadurch gekennzeichnet, daß** sich in Umfangsrichtung des hülsenförmigen Bereichs erstreckende bogenförmige Crimpzonen (51, 52) vorgesehen sind.

5. Anordnung nach einem der vorhergehenden Ansprüche, **dadurch gekennzeichnet, daß** das andere Element (13) mindestens im Fixierbereich eine gemittelte Rauhtiefe Rz von ≥ 10 µm hat.

6. Anordnung zum Einstellen der Relativlage zweier Elemente (13, 67) eines aktiven oder passiven Hör-Implantats und zum Fixieren dieser Elemente in der eingestellten Relativlage, wobei eines der Elemente mindestens im Fixierbereich als zuziehbare Bandschlaufe (69) ausgebildet ist, wobei das andere Element (13) mindestens im Fixierbereich geradlinig stabförmig ausgebildet ist und wobei im fixierten Zustand der schlaufenförmige Teil des einen Elements den geradlinig stabförmigen Teil des anderen Elements über einen Winkel von mindestens 360° umschließt und durch Kraft- und/oder Formschluß auf dem stabförmigen Teil des anderen Elements spielfrei und dauerhaft befestigt ist.

7. Anordnung nach Anspruch 6, **dadurch gekennzeichnet, daß** im fixierten Zustand der schlaufenförmige Teil (69) des einen Elements (67) auf dem stabförmigen Teil des anderen Elements (13) durch Stoffschluß, insbesondere mit chirurgischem Knochenzement (70), zusätzlich gesichert ist.

8. Anordnung nach Anspruch 6 oder 7, **dadurch gekennzeichnet, daß** der schlaufenförmige Teil (69) des einen Elements (67) und der stabförmige Teil des anderen Elements (13) mindestens im Fixierbereich an den einander zugewendeten Flächen aufgerauht sind.

9. Anordnung nach einem der vorhergehenden Ansprüche, **dadurch gekennzeichnet, daß** der hülsen- oder schlaufenförmige Teil (22, 69) des einen Elements (17, 29, 36, 40, 67)) vor dem Fixieren entlang dem stabförmigen Teil des anderen Elements (13) in Axialrichtung verschiebbar ist.

10. Anordnung nach einem der vorhergehenden Ansprüche, **dadurch gekennzeichnet, daß** der hülsen- oder schlaufenförmige Teil (22, 69) des einen Elements (17, 29, 36, 40, 67) vor dem Fixieren gegenüber dem stabförmigen Teil des anderen Elements (13) verdrehbar ist.

11. Anordnung nach einem der vorhergehenden Ansprüche, **dadurch gekennzeichnet, daß** der stabförmige Teil des anderen Elements (13) mindestens im Fixierbereich die Gestalt eines geraden Zylinders oder eines geraden Prismas hat.

12. Anordnung nach einem der vorhergehenden Ansprüche, **dadurch gekennzeichnet, daß** eines der beiden Elemente eine Koppelstange (13) ist, die mit einem aktiv schwingfähigen Teil (12) eines implantierbaren Hörgerätewandlers (10) verbunden ist, und daß das andere der beiden Elemente ein Koppelelement (17, 29, 36, 40, 67) ist, das an die Ossikelkette oder die Perilymphe ankoppelbar ist.

13. Anordnung nach Anspruch 12, **dadurch gekennzeichnet, daß** das Koppelelement (17, 29, 36, 40) eine Crimphülse (22), ein Ankoppelteil (23, 34, 37, 41) für den jeweiligen Ankoppelort im Mittelohr sowie ein die Crimphülse und das Ankoppelteil verbindendes Mittelteil (24, 31, 42) aufweist.

14. Anordnung nach Anspruch 13, **dadurch gekennzeichnet, daß** das Mittelteil (24, 31) verformbar ist.

15. Anordnung nach einem der Ansprüche 12 bis 14, **dadurch gekennzeichnet, daß** das Koppelelement (17) ein um einen Teil der Ossikelkette herumlegbares C-förmiges Ankoppelteil (23) aufweist.

16. Anordnung nach einem der Ansprüche 12 bis 14, **dadurch gekennzeichnet, daß** das Koppelelement (29) eine um einen Teil der Ossikelkette herumlegbare Bandschlaufe (34) aufweist.

17. Anordung nach einem der Ansprüche 12 bis 14, **dadurch gekennzeichnet, daß** das Koppelelement (36) ein auf einen Teil der Ossikelkette aufsetzbares, näherungsweise glockenförmiges Ankoppelteil (37) aufweist.

18. Anordnung nach einem der Ansprüche 12 bis 14, **dadurch gekennzeichnet, daß** das Koppelelement (40) ein über eine Fußplattenperforation in das Vestibulum eintauchendes kolbenförmiges Ankoppelteil (41) aufweist.

19. Anordnung nach einem der Ansprüche 1 bis 11, **dadurch gekennzeichnet, daß** das Implantat als Total- oder Teilersatz (58, 59) für die Ossikelkette ausgebildet ist.

20. Anordnung nach einem der vorhergehenden Ansprüche, **dadurch gekennzeichnet, daß** das eine Element (17, 29, 36, 40) mindestens im Fixierbereich aus Gold, insbesondere Feingold, Platin, insbesondere Reinplatin, oder Silber oder aus Legierungen der genannten Metall besteht.

21. Anordnung nach einem der vorhergehenden Ansprüche, **dadurch gekennzeichnet, daß** das andere Element (13) mindestens im Fixierbereich aus Titan, insbesondere Reintitan, Platin, Niob oder Tantal oder aus Legierungen der genannten Metalle oder aus implantierbarem Edelstahl oder keramischem Werkstoff, insbesondere Aluminiumoxid, besteht.

## Claims

1. Arrangement for adjusting the relative position of two elements of an active or a passive hearing implant (10, 58, 59) and for fixing these elements in the adjusted relative position; wherein one (17, 29, 36, 40, 61) of the elements, at least in a fixing region thereof, is sleeve-shaped and is plastically cold-deformable by means of a crimping tool (45); wherein the other one (13, 64) of the elements, at least in a fixing region thereof, is rod-shaped, is provided with a rough surface and is resistant to being plastically cold-deformed by the crimping force applied by means of the crimping tool (45); and wherein, in a fixed state, the sleeve-shaped part of the one of the elements is permanently attached on the rod-shaped part of the other one of the elements without play by having been cold flow deformed by the crimping force.

2. Arrangement as claimed in claim 1, **characterized in that** the crimp angle (α) is 90° ± 15°.

3. Arrangement as claimed in claim 1 or 2, **characterized in that** there are two diametrically opposite crimp zones (51, 52).

4. Arrangement as claimed in any one of the preceding claims, **characterized in that** arc-shaped crimp zones (51, 52) are provided that extend in a peripheral direction of the sleeve-shaped part.

5. Arrangement as claimed in any one of the preceding claims, **characterized in that** the other one (13) of the elements, at least in the fixing region thereof, has an averaged surface roughness Rz of at least 10 µm.

6. Arrangement for adjusting the relative position of two elements (13, 67) of an active or a passive hearing implant and for fixing these elements in the adjusted position; wherein one of the elements, at least in a fixing region thereof, is in the form of a tightenable belt loop (69); wherein the other one (13) of the elements, at least in a fixing region thereof, is straight rod-shaped; and wherein, in the fixed state, the loop-shaped part of the one of the elements extends around the straight rod-shaped part of the other one of the elements over an angle of at least 360° and is permanently attached on the rod-shaped part without play by a force-fit or a form-fit type connection.

7. Arrangement as claimed in claim 6, **characterized in that** the loop-shaped part (69) of the one (67) of the elements, in the fixed state, is additionally secured on the rod-shaped part of the other one (13) of the elements by an adhesive bond, particularly with surgical bone cement (70)

8. Arrangement as claimed in claim 6 or 7, **characterized in that** the loop-shaped part (69) of the one (67) of the elements and the rod-shaped part of the other one (13) of the elements are roughened on facing surfaces thereof, at least in the fixing region.

9. Arrangement as claimed in any one of the preceding claims, **characterized in that** the sleeve- or loop-shaped part (22, 69) of the one (17, 29, 36, 40, 67) of the elements is axially movable along the rod-shaped part of the other one (13) of the elements prior to fixing thereof.

10. Arrangement as claimed in any one of the preceding claims, **characterized in that** the sleeve- or loop-shaped part (22, 69) of the one (17, 29, 36, 40, 67) of the elements is rotatable relative to the rod-shaped part of the other one (13) of the elements prior to fixing thereof.

11. Arrangement as claimed in any one of the preceding claims, **characterized in that** the rod-shaped part of the other one (13) of the elements, at least in the fixing region, is in the shape of a straight cylinder or of a straight prism.

12. Arrangement as claimed in any one of the preceding claims, **characterized in that** one of the two elements is a coupling rod (13) which is joined with an actively oscillatory part (12) of an implantable hearing aid converter (10), and that the other one of the two elements is a coupling element (17, 29, 36, 40, 67) which is adapted for being coupled to the ossicle chain or to the perilymph.

13. Arrangement as claimed in claim 12, **characterized in that** the coupling element (17, 29, 36, 40) comprises a crimp sleeve (22), a coupling part (23, 34, 37, 41) for the respective coupling site in the middle ear, and a middle part (24, 31, 42) which connects the crimp sleeve and the coupling part together.

14. Arrangement as claimed in claim 13, **characterized in that** the middle part (24, 31) is deformable.

15. Arrangement as claimed in any one of claims 12 to 14, **characterized in that** the coupling element (17) has a C-shaped coupling part (23) which is positionable around a part of the ossicle chain.

16. Arrangement as claimed in any one of claims 12 to 14, **characterized in that** the coupling element (29) has a belt loop (34) which is positionable around a part of the ossicle chain.

17. Arrangement as claimed in any one of claims 12 to 14, **characterized in that** the coupling element (36) has a roughly bell-shaped coupling part (37) which is positionable on a part of the ossicle chain.

18. Arrangement as claimed in any one of claims 12 to 14, **characterized in that** the coupling element (40) has a piston-shaped coupling part (41) which is insertable into the vestibulum via a footplate perforation.

19. Arrangement as claimed in any one of claims 1 to 11, **characterized in that** the implant is a partial or total replacement (58, 59) for the ossicle chain.

20. Arrangement as claimed in any one of the preceding claims, **characterized in that** the first one (17, 29, 36, 40) of the elements, at least in the fixing region, is made of gold, particularly fine gold, platinum, particularly pure platinum, or silver, or of alloys of said metals.

21. Arrangement as claimed in any one of the preceding claims, **characterized in that** the other one (13) of the elements, at least in the fixing region, is made of titanium, particularly pure titanium, platinum, niobium, or tantalum or of alloys of said metals, or of implantable high quality steel or a ceramic material, particularly alumina.

## Revendications

1. Dispositif destiné au réglage de la position relative de deux éléments d'un implant auditif actif ou passif (10, 58, 59), et à la fixation de ces éléments dans la position relative réglée, l'un des éléments (17, 29, 36, 40, 61) étant réalisé en forme de douille, au moins dans la zone de fixation, et pouvant être déformé plastiquement à froid au moyen d'un outil de sertissage (45), l'autre élément (13, 64) étant réalisé en forme de barre, au moins dans la zone de fixation, étant doté d'une surface rugueuse, et ne pouvant pas être déformé plastiquement à froid sous l'effet de la force de sertissage exercée au moyen de l'outil de sertissage (45) et, à l'état fixé, la partie en forme de douille de l'un des éléments, déformée par fluage à froid par la force de sertissage, étant fixée sans jeu et durablement sur la partie en forme de barre de l'autre élément.

2. Dispositif selon la revendication 1, **caractérisé en ce que** l'angle de sertissage (α) est de 90° ± 15°.

3. Dispositif selon la revendication 1 ou 2, **caractérisé en ce qu'**il est prévu deux zones de sertissage (51, 52) situées diamétralement à l'opposé l'une de l'autre.

4. Dispositif selon l'une des revendications précédentes, **caractérisé en ce qu'**il est prévu des zones de sertissage (51, 52) cintrées s'étendant dans la direction périphérique de la zone en forme de douille.

5. Dispositif selon l'une des revendications précédentes, **caractérisé en ce que** l'autre élément (13) possède une profondeur de rugosité moyenne Rz = 10 µm, au moins dans la zone de fixation.

6. Dispositif destiné au réglage de la position relative de deux éléments (13, 67) d'un implant auditif actif ou passif, et à la fixation de ces éléments dans la position relative réglée, l'un des éléments étant réalisé sous la forme d'une boucle de bande (69) pouvant être resserrée, au moins dans la zone de fixation, l'autre élément (13) étant réalisé en forme de barre rectiligne, au moins dans la zone de fixation et, à l'état fixé, la partie en forme de boucle de l'un des éléments enserrant la partie en forme de barre rectiligne de l'autre élément sur un angle d'au moins 360°, et étant fixé par adhérence de force et/ou de forme, sans jeu et durablement, sur la partie en forme de barre de l'autre élément.

7. Dispositif selon la revendication 6, **caractérisé en ce qu'**à l'état fixé, la partie en forme de boucle (69) de l'un des éléments (67) est sécurisée de façon supplémentaire par adhérence de matière, notamment au moyen de ciment osseux chirurgical (70), sur la partie en forme de barre de l'autre élément (13).

8. Dispositif selon la revendication 6 ou 7, **caractérisé en ce que** les surfaces orientées l'une vers l'autre de la partie en forme de boucle (69) de l'un des éléments (67), et de la partie en forme de barre de l'autre élément (13), sont rugueuses, au moins dans la zone de fixation.

9. Dispositif selon l'une des revendications précédentes, **caractérisé en ce que**, avant la fixation, la partie en forme de douille ou de boucle (22, 69) de l'un des éléments (17, 29, 36, 40, 67) peut être déplacée en translation dans la direction axiale le long de la partie en forme de barre de l'autre élément (13).

10. Dispositif selon l'une des revendications précédentes, **caractérisé en ce que**, avant la fixation, la partie en forme de douille ou de boucle (22, 69) de l'un des éléments (17, 29, 36, 40, 67) peut être tournée par rapport à la partie en forme de barre de l'autre élément (13).

11. Dispositif selon l'une des revendications précédentes, **caractérisé en ce que** la partie en forme de barre de l'autre élément (13) a la forme d'un cylindre droit ou d'un prisme droit, au moins dans la zone de fixation.

12. Dispositif selon l'une des revendications précédentes, **caractérisé en ce que** l'un des deux éléments est une tige de couplage (13), qui est reliée à une partie (12) susceptible de vibrer activement d'un convertisseur (10) de prothèse auditive implantable, et **en ce que** l'autre des deux éléments est un élément de couplage (17, 29, 36, 40, 67) pouvant être couplé à la chaîne des osselets ou à la périlymphe.

13. Dispositif selon la revendication 12, **caractérisé en ce que** l'élément de couplage (17, 29, 36, 40) comporte une douille de sertissage (22), une pièce d'accouplement (23, 34, 37, 41) pour l'emplacement de couplage respectif dans l'oreille moyenne, ainsi qu'une partie médiane (24, 31, 42) reliant la douille de sertissage et la pièce d'accouplement.

14. Dispositif selon la revendication 13, **caractérisé en ce que** la partie médiane (24, 31) peut être déformée.

15. Dispositif selon l'une des revendications 12 à 14, **caractérisé en ce que** l'élément de couplage (17) comporte une pièce d'accouplement (23) en forme de C pouvant être placée autour d'une partie de la chaîne des osselets.

16. Dispositif selon l'une des revendications 12 à 14, **caractérisé en ce que** l'élément de couplage (29) comporte une boucle de bande (34) pouvant être placée autour d'une partie de la chaîne des osselets.

17. Dispositif selon l'une des revendications 12 à 14, **caractérisé en ce que** l'élément de couplage (36) comporte une pièce d'accouplement (37) approximativement en forme de cloche, pouvant être emboîtée sur une partie de la chaîne des osselets.

18. Dispositif selon l'une des revendications 12 à 14, **caractérisé en ce que** l'élément de couplage (40) comporte une pièce d'accouplement (41) en forme de piston, qui s'engage dans le vestibule par l'intermédiaire d'une perforation de la plaque de base.

19. Dispositif selon l'une des revendications 1 à 11, **caractérisé en ce que** l'implant est réalisé en tant que substitut total ou partiel (58, 59) de la chaîne des osselets.

20. Dispositif selon l'une des revendications précédentes, **caractérisé en ce que**, au moins dans la zone de fixation, l'un des éléments (17, 29, 36, 40) est en or, notamment en or fin, en platine, notamment en platine pur, ou en argent ou en alliages de ces métaux.

21. Dispositif selon l'une des revendications précédentes, **caractérisé en ce que**, au moins dans la zone de fixation, l'autre élément (13) est en titane, notamment en titane pur, en platine, en niobium ou en tantale ou en alliages de ces métaux, ou en acier spécial ou matériau céramique implantable, notamment en oxyde d'aluminium.
